# EUROPEAN PATENT SPECIFICATION

(11) **EP 0 907 364 B1**
(45) Date of publication and mention of the grant of the patent: **14.08.2002**
(21) Application number: 97924103.1
(22) Date of filing: 27.05.1997
(51) Int. Cl.: A61K 31/55, A61K 9/20, A61P 25/18

(54) **SUSTAINED RELEASE PHARMACEUTICAL COMPOSITIONS COMPRISING A DIBENZOTHIAZEPINE DERIVATIVE**
ARZNEIMITTEL MIT VERZÖGERTER FREISETZUNG, DAS EIN DIBENZOTHIAZEPINDERIVAT ENTHÄLT
COMPOSITIONS PHARMACEUTIQUES A LIBERATION PROLONGEE CONTENANT UN DERIVE DE DIBENZOTHIAZEPINE

(30) Priority: 31.05.1996 GB 9611328
(43) Date of publication of application: 14.04.1999
(73) Proprietor: AstraZeneca AB, 151 85 Södertälje (SE)
(72) Inventor: PARIKH, Bhavnish, Vinod, Wilmington, DE 19897 (US); TIMKO, Robert, Joseph, Wilmington, DE 19897 (US); ADDICKS, William, Joseph, Morgantown, WV 26505 (US)
(74) Representative: Bill, Kevin
(86) International application number: GB9701432
(87) International publication number: WO9745124

(56) References cited:
- EP-A- 0 240 228
- EP-A- 0 282 236

## Description

The present invention relates to a pharmaceutical composition and more particularly to a sustained release pharmaceutical composition comprising 11-[4-[2-(2-hydroxyethoxy)ethyl]-1-piperazinyl]dibenzo[b,f][1,4]thiazepine or a pharmaceutically acceptable salt thereof.

It is desirable in the treatment of a number of diseases, both therapeutically and prophylactically, to provide the active pharmaceutical ingredient in a sustained release form. Desirably the sustained release provides a generally uniform and constant rate of release over an extended period of time which achieves a stable and desired blood (plasma) level of the active ingredient without the need for frequent administration of the medicament.

While there are numerous sustained release formulations known in the art which utilize gelling agents, such as hydroxypropyl methylcelluloses, it has been found to be difficult to formulate sustained release formulations of soluble medicaments and gelling agents, such as hydroxypropyl methylcellulose, for several reasons. First of all, active ingredients which are soluble in water tend to generate a sustained release product which is susceptible to a phenomenon known as dose dumping. That is, release of the active ingredient is delayed for a time but once release begins to occur the rate of release is very high. Moreover, fluctuations tend to occur in the plasma concentrations of the active ingredient which increases the likelihood of toxicity. Further, some degree of diurnal variation in plasma concentration of the active ingredient has also been observed. Finally, it has been found to be difficult to achieve the desired dissolution profiles or to control the rate of release of the soluble medicament.

Accordingly, a need exists for sustained release formulations of soluble medicaments, such as, 11-[4-[2-(2-hydroxyethoxy)ethyl]-1-piperazinyl]-dibenzo[b,f][1,4]thiazepine or a pharmaceutically acceptable salt, which overcome, or at least alleviate, one or more of the above described difficulties and which further provide the advantageous property of allowing the active medicament to be administered less frequently, e.g. once a day, while achieving blood (plasma) levels similar to those attained by administering smaller doses of the medicament more frequently, e.g. two or more times daily.

Figure 1 shows the release (dissolution) profiles of the sustained release formulations of Examples 8, 9 and 10 which are obtained by immersing a suitable tablet in 750 mL of 0.1 N HCl for 2 hours at 37°C and a speed of 100 rpm and then adding 250 mL of 0.2 M sodium phosphate buffer to the dissolution media to afford a pH of 6.2.

Figure 2 shows the plasma concentration versus time profiles of the active ingredient for the sustained release formulations of examples 1 and 2 and the immediate release formulation of example 12.

The compound, 11-[4-[2-(2-hydroxyethoxy)ethyl]- 1-piperazinyl]-dibenzo[b,f][1,4]thiazepine (see Formula I below), and its pharmaceutically acceptable salts exhibit useful antidopaminergic activity and may be used, for 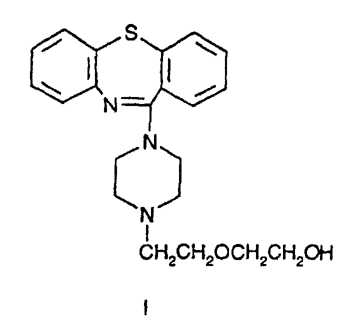 example, as an antipsychotic agent (for example, for the management of the manifestations of psychotic disorders) or as a treatment for hyperactivity. It is a compound of particular interest since it may be used as an antipsychotic agent with a substantial reduction in the potential to cause side effects such as acute dystonia, acute dyskinesia, pseudo-Parkinsonism and tardive dyskinesia which side-effects may result from the use of other antipsychotics or neuroleptics.

The preparation, physical properties and beneficial pharmacological properties of 11-[4-[2-(2- hydroxyethoxy)ethyl]-1-piperazinyl]dibenzo[b,f][1,4]-thiazepine, and its pharmaceutically acceptable salts are described in published European Patents EP 240,228 and 282,236 as well as in U.S. Patent 4,879,288.

According to the present invention there is provided a sustained release formulation comprising a gelling agent, preferably hydroxypropyl methylcellulose, and 11-[4-[2-(2-hydroxyethoxy)ethyl]-1-piperazinyl]dibenzo-[b,f][1,4]thiazepine, or a pharmaceutically acceptable salt thereof, together with one or more pharmaceutically acceptable excipients. Preferably, the sustained release formulation comprises a hydrophilic matrix comprising a gelling agent, preferably hydroxypropyl methylcellulose, and 11-[4-[2-(2-hydroxyethoxy)ethyl]-1-piperazinyl]dibenzo-[b,f][1,4]thiazepine, or a pharmaceutically acceptable salt thereof, together with one or more pharmaceutically acceptable excipients.

The term gelling agent as used herein means any substance, particularly a hydrophilic substance, which forms a gel when in contact with water and thus includes such substances as hydroxypropyl methylcellulose, hydroxypropylcellulose, hydroxymethylcellulose, hydroxyethylcellulose, hydroxypropyl ethylcellulose, methylcellulose, ethylcellulose, carboxyethylcellulose, carboxymethyl hydroxyethylcellulose, carbomer, sodium carboxymethylcellulose, polyvinylpyrrolidone, and the like, or mixtures thereof. The gelling agent is preferably hydroxypropyl methylcellulose.

The amount of gelling agent, preferably hydroxypropyl methylcellulose, is preferably selected such that the active ingredient is released from the formulation, in a controlled fashion, over a period of 4 hours or longer, preferably over a period of 8 hours or longer and in particular over a period of between 8 and 24 hours, that is so that at least 60% of the active ingredient has been released at the end of this period.

The gelling agent, preferably hydroxypropyl methylcellulose, is conveniently present in about 5 to 50% (by weight), more conveniently about 5 to 40%, most conveniently about 8 to 35% and in particular about 10 to 35%. It is generally preferred that the gelling agent, preferably hydroxypropyl methylcellulose, is present in about 10 to 30%, more preferably about 15 to 30%.

The hydroxypropyl methylcellulose may contain more than one grade of polymer and is commercially available under several trademarks, e.g. METHOCEL® E, F, J and K from the Dow Chemical Company, U.S.A. and METALOSE™ SH from Shin-Etsu, Ltd., Japan. The various grades available under a given trademark represent differences in methoxy and hydroxypropoxy content as well as in viscosity. The methoxy content ranges from 16.5 to 30% by weight, the hydroxypropoxy content ranges from 4 to 32% by weight and the viscosities of a 2% aqueous solution at 20°C range from 3 cps to 100,000 cps. For example, the hydroxypropyl methylcellulose preferably comprises (a) a polymer with a viscosity of about 40 to 60 cps (in particular about 50 cps), a methoxy content of about 28 to 30% by weight and a hydroxypropoxy content of from about 7 to less than 9% by weight; or (b) a polymer with a viscosity of about 3,500 to 5,600 cps (in particular about 4,000 cps), a methoxy content of about 28 to 30% by weight and a hydroxypropoxy content of about 7 to 12% by weight; or (c) a polymer with a viscosity of about 80 to 120 cps (in particular about 100 cps), a methoxy content of about 19 to 24% by weight and a hydroxypropoxy content of from about 7 to less than 9% by weight; or (d) a polymer with a viscosity of about 3500 to 5600 cps (in particular about 4,000 cps), a methoxy content of about 19 to 24% by weight and a hydroxypropoxy content of about 7 to 12% by weight, or mixtures thereof. More preferably, the hydroxypropyl methylcellulose is selected from the group consisting of (a) - (d) or mixtures thereof as described above with the proviso that if the formulation contains a hydroxypropyl methylcellulose described under (d) above the total amount of hydroxypropyl methylcellulose present in the formulation must be greater than 25.8% by weight.

In one embodiment the hydroxypropyl methylcellulose comprises 8 to 12% of a polymer having a viscosity of about 4,000 cps, and preferably about 5 to 10%. In a further embodiment hydroxypropyl methylcellulose comprises 10 to 35% of a polymer having a viscosity of about 50 cps, and preferably about 10 to 15%.

In a specific embodiment the hydroxypropyl methylcellulose comprises 15% of a polymer having a viscosity of about 50 cps, and optionally about 5% of a hydroxypropyl methylcellulose polymer having a viscosity of about 4,000 cps.

In particular the 11-[4-[2-(2-hydroxyethoxy)-ethyl]-1-piperazinyl]-dibenzo[b,f][1,4]thiazepine, or pharmaceutically acceptable salt thereof (preferably the hemifumarate salt), is present in about 10 to 90% by weight, preferably about 20 to 80% by weight, more preferably about 35 to 65% by weight, most preferably about 40 to 60% by weight and especially about 43.2 to 57.6% by weight.

The formulation will, in general, contain one or more excipients. Such excipients will include diluents such as lactose, microcrystalline cellulose, dextrose, mannitol, sucrose, sorbitol, gelatin, acacia, dicalcium phosphate, tricalcium phosphate, monocalcium phosphate, sodium phosphate, sodium carbonate and the like, preferably lactose and microcrystalline cellulose; lubricants such as stearic acid, zinc, calcium or magnesium stearate and the like, preferably magnesium stearate; binders such as sucrose, polyethylene glycol, povidone (polyvinylpyrrolidone), corn or maize starch, pregelatinized starch and the like, preferably povidone (polyvinylpyrrolidone); colorants such as ferric oxides, FD & C dyes, lakes and the like; flavoring agents; and pH modifiers which include suitable organic acids or alkali metal (e.g. lithium, sodium or potassium) salts thereof, such as benzoic acid, citric acid, tartaric acid, succinic acid, adipic acid and the like or the corresponding alkali metal salts thereof, preferably the alkali metal salts of such acids and in particular the sodium salt of citric acid (i.e. sodium citrate). The excipient(s) will, in general, be present in about 10 to 90% by weight, preferably about 20 to 80% by weight, more preferably about 20 to 45% by weight, most preferably about 20 to 40% by weight and especially about 22.4 to 36.8% by weight. The formulation preferably may contain one or more pharmaceutically acceptable excipients selected from the group consisting of microcrystalline cellulose, lactose, magnesium stearate, sodium citrate and povidone. In particular, the formulation may contain one or more of (a) microcrystalline cellulose, preferably in the amount of about 4 to 20% by weight, (b) lactose, preferably in the amount of about 5 to 20% by weight, (c) magnesium stearate, preferably in the amount of about 1 to 3% by weight, (d) about 10 to 30% by weight, preferably about 12.5 to 25% and in particular about 12.5% by weight of sodium citrate, and (e) about 1 to 15% by weight, preferably about 4 to 6% by weight and in particular about 5% by weight of povidone (polyvinylpyrrolidone).

According to the present invention there is also provided a sustained release formulation comprising a gelling agent, preferably hydroxypropyl methylcellulose, and 11-[-4-[2-(2-hydroxyethoxy)ethyl]-1-piperazinyl]dibenzo-[b,f][1,4]thiazepine, or a pharmaceutically acceptable salt thereof, together with one or more pharmaceutically acceptable excipients wherein one of the excipients is a pH modifier.

According to the present invention there is also provided a sustained release formulation comprising 11-[4-[2-(2-hydroxyethoxy)ethyl]-1-piperazinyl)-dibenzo[b,f][1,4]thiazepine, or a pharmaceutically acceptable salt thereof, as active ingredient and 5 to 40% of hydroxypropyl methylcellulose, together with one or more pharmaceutically acceptable excipients.

According to the present invention there is also provided a sustained release formulation comprising about 35 to 65% of 11-[4-[2-(2-hydroxyethoxy)-ethyl]-1-piperazinyl]dibenzo[b,f][1,4]thiazepine, or a pharmaceutically acceptable salt thereof, as active ingredient and about 5 to 40% by weight of hydroxypropyl methylcellulose, together with one or more pharmaceutically acceptable excipients.

According to the present invention there is also provided a sustained release formulation comprising about 35 to 65% of 11-[4-[2-(2-hydroxyethoxy)-ethyl]-1-piperazinyl]dibenzo[b,f][1,4]thiazepine, or a pharmaceutically acceptable salt thereof, as active ingredient and about 15 to 30% of hydroxypropyl methylcellulose, together with about 20 to 45% of one or more pharmaceutically acceptable excipients.

According to the present invention there is also provided a sustained release formulation comprising about 35 to 65% of 11-[4-[2-(2-hydroxyethoxy)-ethyl]-1-piperazinyl]-dibenzo[b,f][1,4]thiazepine as active ingredient, or a pharmaceutically acceptable salt thereof, about 5 to 40% by weight of hydroxypropyl methylcellulose, about 4 to 12% microcrystalline cellulose, about 8 to 20% lactose and the remainder being one or more further pharmaceutically acceptable excipients. Such further excipients may include components which act as a lubricant (for example, magnesium stearate) during the manufacture of the formulation or dosage form.

According to the present invention there is also provided a sustained release formulation comprising about 5 to 40% by weight of a hydroxypropyl methylcellulose selected from the group consisting of (a) a hydroxypropyl methylcellulose having a viscosity of about 40 to 60 cps, a methoxy content of about 28 to 30% by weight and a hydroxypropoxy content of from about 7 to less than 9% by weight, (b) a hydroxypropyl methylcellulose having a viscosity of about 3,500 to 5,600 cps, a methoxy content of about 28 to 30% by weight and a hydroxypropoxy content of about 7 to 12% by weight, (c) a hydroxypropyl methylcellulose having a viscosity of about 80 to 120 cps, a methoxy content of about 19 to 24% by weight and a hydroxypropoxy content of from about 7 to less than 9% by weight and (d) a hydroxypropyl methylcellulose having a viscosity of about 3,500 to 5,600 cps, a methoxy content of about 19 to 24% by weight and a hydroxypropoxy content of about 7 to 12% by weight, or mixtures thereof; about 35 to 65% by weight of 11-[4-[2-(2-hydroxyethoxy)ethyl]-1-piperazinyl]-dibenzo[b,f][1,4]thiazepine or a pharmaceutically acceptable salt thereof; and about 20 to 45% by weight of one or more pharmaceutically acceptable excipients; with the proviso that if the formulation contains a hydroxypropyl methylcellulose described under (d) above the total amount of hydroxypropyl methylcellulose present in the formulation must be greater than 25.8% by weight.

Other formulations within the ambit of this latter group are those comprising about 8 to 35% by weight of a hydroxypropyl methylcellulose selected from the group consisting of (a) a hydroxypropyl methylcellulose having a viscosity of about 40-60 cps, a methoxy content of about 28 to 30% by weight and a hydroxypropoxy content of about 7 to less than 9% by weight, (b) a hydroxypropyl methylcellulose having a viscosity of about 3,500 to 5,600 cps, a methoxy content of about 28 to 30% by weight and a hydroxypropoxy content of about 7 to 12% by weight, (c) a hydroxypropyl methylcellulose having a viscosity of about 80 to 120 cps, a methoxy content of about 19 to 24% by weight and a hydroxypropoxy content of about 7 to less than 9% by weight and (d) a hydroxypropyl methylcellulose having a viscosity of about 3,500 to 5,600 cps, a methoxy content of about 19 to 24% by weight and a hydroxypropoxy content of about 7 to 12% by weight or mixtures thereof; about 35 to 65% by weight of 11-[4-[2'(2-hydroxyethoxy)ethyl]-1-piperazinyl]dibenzo[b,f][1,4]thiazepine or a pharmaceutically acceptable salt thereof; and about 20 to 45% by weight of one or more pharmaceutically acceptable excipients.

Still other formulations within the ambit of this latter group are those comprising about 10 to 30% by weight of a hydroxypropyl methylcellulose selected from the groups (a) - (d) or mixtures thereof as described above; about 40 to 60% by weight of 11-[4-[2-(2-hydroxyethoxy)ethyl]-1-piperazinyl]dibenzo[b,f][1,4]-thiazepine or a pharmaceutically acceptable salt thereof; and about 20 to 40% by weight of one or more pharmaceutically acceptable excipients.

Preferred formulations within this latter group are those comprising about 15 to 30% by weight of a hydroxypropyl methylcellulose selected from the groups (a) - (d) or mixtures thereof as described above; about 43.2 to 57.6% by weight of 11-[4-[2-(2-hydroxyethoxy)ethyl]-1-piperazinyl]dibenzo[b,f]-[1,4]thiazepine or a pharmaceutically acceptable salt thereof; and about 22.4 to 36.8% by weight of one or more pharmaceutically acceptable excipients.

Particularly preferred formulations within this latter group are those comprising about 15 to 30% by weight of a hydroxypropyl methylcellulose selected from the groups (a) - (d) or mixtures thereof as described above; about 43.2 to 57.6% by weight of 11-[4-[2-(2-hydroxyethoxy)ethyl]-1-piperazinyl]dibenzo[b,f]-[1,4]thiazepine or a pharmaceutically acceptable salt thereof; and about 22.4 to 36.8% by weight of one or more pharmaceutically acceptable excipients selected from the group consisting of (a) about 4 to 12% by weight of microcrystalline cellulose, (b) about 5 to 20% by weight of lactose, (c) about 1 to 3% by weight of magnesium stearate, (d) about 10 to 30% by weight of sodium citrate and (e) about 1 to 15% by weight of povidone (polyvinylpyrrolidone).

In the above-described formulations the 11-[4-[2-(2-hydroxyethoxy)ethyl]-1-piperazinyl]dibenzo[b,f][1,4]-thiazepine is preferably in the form of a hemifumarate salt which form has an equilibrium solubility in water at 20°C of 3.29 mg/mL.

Formulations of particular interest include those described in the accompanying Examples and so formulations substantially as defined in the accompanying Examples are provided as a further feature of the present invention.

As mentioned above, the compound 11-[4-[2-(2-hydroxyethoxy)-ethyl]-1-piperazinyl]dibenzo[b,f]]1,4]-thiazepine, and its pharmaceutically acceptable salts, exhibit useful antidopaminergic activity and may be used, for example, as an antipsychotic agent (for example, for the management of the manifestations of psychotic disorders) or as a treatment for hyperactivity. Thus, the present invention also provides a method of treating psychotic states, for example psychosis, in a warm-blooded animal, such as man, which comprises administering an effective amount of the formulation of the present invention to said warm-blooded animal.

The formulation according to the present invention can be used in a method of treating hyperactivity in a warm-blooded animal which comprises administering to said warm-blooded animal an effective amount of a formulation of the present invention.

The formulations of the present invention may be prepared by conventional technology well known to those skilled in the art such as wet granulation, direct compression, dry compaction (slugging) and the like. Thus, for example, the active ingredient 11-[4-[2-(2-hydroxyethoxy)ethyl]-1-piperazinyl]-dibenzo[b,f]-[1,4]thiazepine, or a pharmaceutically acceptable salt thereof, a gelling agent, preferably hydroxypropyl methylcellulose, and other excipients are mixed together to form the sustained release formulations of the present invention. Preferably the active ingredient 11-[4-[2-(2-hydroxyethoxy)ethyl]-1-piperazinyl]-dibenzo[b,f][1,4]thiazepine, or a pharmaceutically acceptable salt thereof, a gelling agent, preferably hydroxypropyl methylcellulose, and other excipients are mixed together to form a mixture suitable for compressing into tablets, which mixture is then compressed to form tablets or is filled into capsules

The mixing process is preferably carried out by mixing the components, wet granulating the mixed components, drying the mixture, milling the dried mixture, blending the mixture with a lubricant such as magnesium stearate and compressing the blended mixture to form tablets or filling the blended mixture into capsules.

A preferred process for preparing the formulations of the invention comprises the following steps:
(a) mixing 11-[4-[2-(2-hydroxyethoxy)ethyl]-1-piperazinyl]dibenzo[b,f][1,4]-thiazepine, or a pharmaceutically acceptable salt thereof, a gelling agent, preferably hydroxypropyl methylcellulose, and other excipients;
(b) wet granulating the mixed components;
(c) drying the mixture;
(d) milling the dried mixture;
(e) blending the mixture with a lubricant such as magnesium stearate; and
(f) compressing the blended mixture to form tablets.

The dosage forms may be coated with one or more coatings as is well known in the art such as, for example, shellac, zein, hydroxypropyl cellulose, hydroxypropyl methylcellulose, ethyl cellulose, polymethacrylates, polyvinyl acetate phthalate, cellulose acetate phthalate, triacetin, dibutyl sebacate, a mixture of polyethylene glycol, titanium dioxide and hydroxypropyl methylcellulose, and the like.

The sustained release properties of the formulation of the present invention may be demonstrated by monitoring the dissolution of the active ingredient. The dissolution of the active ingredient may be monitored using standard procedures well known to those skilled in the art (e.g. the dissolution test procedures, such as the Rotating Basket Method (Apparatus I) or Paddle Method (Apparatus II), disclosed in the U.S. Pharmacopeia (USP)). Such procedures include those in which the formulation is immersed in an aqueous medium such as water or hydrochloric acid and aliquots of the medium are withdrawn at various time points over a period of 24 hours. The aliquots are analyzed using high pressure liquid chromatography (HPLC) with UV detection to determine the concentration of dissolved active ingredient using standard methodology. In a particular example a tablet is immersed in about 900 mL of water and the dissolution profile determined. In another particular example, the dissolution profile is determined by the Rotating Basket method by immersing a tablet in 750 mL of 0.1N HCl for 2 hours at a speed of 100 rpm and then adding 250 mL of 0.2 M phosphate buffer to the dissolution media to afford a pH of 6.2.

The formulation preferably releases the active ingredient in a controlled manner over a period of up to about 8 hours or longer. For example, the formulation described in Example 2 below released about 90% of the active ingredient over 16 hours, and the formulation described in Example 1 released about 90% of the active ingredient over a period of 8 hours.

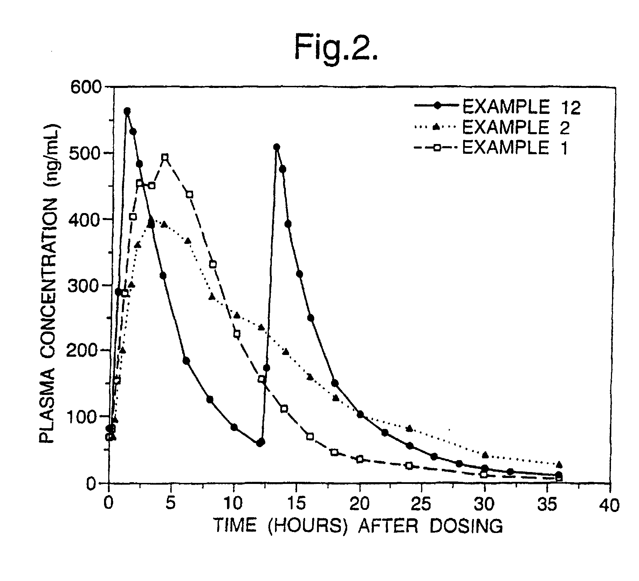

The plasma concentration versus time profiles of the active ingredient illustrated in Figure 2 were obtained utilizing the following procedure. Thirty-two patients were assigned to either Group A or Group B with 16 patients in each group. After a 2-day drug-free period (days 1 and 2), all patients were given oral doses of the immediate release formulation of example 12 twice daily for a 9-day period (days 3 through 11) with fixed step-wise increases in dose from 25 to 200 mg. Starting on day 12, patients began a randomized treatment sequence within their respective groups (Group A or B). Group A patients followed a treatment sequence that included one of each of the following formulations of the active ingredient administered according to the sequence randomized; two 100 mg tablets of the immediate release formulation of example 12 while fasting administered every 12 hours (Treatment 1), one 400 mg tablet of the formulation of example 2 while fasting (Treatment 2) and one 400 mg tablet of the formulation of example 2 with a meal (Treatment 3). Group B patients were randomized to a treatment sequence that included one of each of the following formulations of the active ingredient administered according to the sequence randomized: two 100 mg tablets of the immediate release formulation of example 12 while fasting administered every 12 hours (Treatment 1), one 400 mg tablet of the formulation of example 1 while fasting (Treatment 4) and one 400 mg tablet of the formulation of example 1 with a meal (Treatment 5). On days 12, 16 and 20 patients received trial treatment according to their assigned treatment sequences. On the evenings of days 13 and 17, patients received 200 mg doses of the immediate release formulation of example 12 and on days 14, 15, 18 and 19 the patients received 200 mg dose of the immediate release formulation of example 12 twice daily. Blood samples were taken from each subject on days 3, 10, 11, 14, 15, 18 and 19 before the morning dose. On days, 12, 16 and 20 blood samples were taken from each subject immediately before dose administration and at specified time intervals from immediately after dose administration to 36 hours after dose administration. The concentration of the active ingredient in the blood samples was quantified using liquid-liquid extraction and high performance liquid chromatography with ultraviolet absorbance detection. The plasma concentration of the active ingredient over time profiles for the formulations of examples 1 (n = 11), 2 (n = 10) and 12 (n = 10 for Group A and 12 for Group B) are illustrated in Figure 2 and Table A summarizes the mean area under the curve (AUC) values for a 24 hour dosing interval and the mean maximum blood concentration (Cₘₐₓ) values for each of the examples.

**Table A**

| | **Group A** | | **Group B** | |
|---|---|---|---|---|
| **Example No.** | **AUC**_{**0-24**} | **C**_{**max**} | **AUC**_{**0-24**} | **C**_{**max**} |
| 1 | - | - | 4886 | 565 |
| 2 | 5609 | 433 | - | - |
| 12 | 5347 | 703 | 4818 | 563 |

The dose of the compound of the present invention which is administered will necessarily be varied according to principles well known in the art taking account of the route of administration, the duration of treatment, the severity of the psychotic condition, the size and age of the patient, the potency of the active component and the patient's response thereto. An effective dosage amount of the active component can thus readily be determined by the clinician after a consideration of all criteria and using his best judgment on the patient's behalf. In general, the compound will be administered to a warm blooded animal (such as man) so that an effective dose is received, generally a daily dose in the range of about 0.01 to about 40 mg/kg body weight. For example, when administered orally, it is generally administered in the range of about 0.1 to about 40 mg/kg body weight. Preferably, the compound of the present invention is administered in about a 25, 50, 200, 300 or 400 mg strength.

The formulation of the present invention will, in general, be in the form of a unit dosage form, and, in particular, the formulation will be in the form of a tablet.

It will be apparent to those skilled in the art that the formulation can be co-administered with other therapeutic or prophylactic agents and/or medicaments that are not medically incompatible therewith. The formulation of the present invention does not, in general, show any indication of overt toxicity in laboratory test animals at several multiples of the minimum effective dose of the active ingredient.

The invention is further illustrated by the following non-limiting Examples in which temperatures are expressed in degrees Celsius. The compound 11-[4-[2-(2-hydroxyethoxy)ethyl]-1-piperazinyl]dibenzo[b,f]]1,4]-thiazepine, and its pharmaceutically acceptable salts, may be prepared as described in published European Patents EP 240,228 or 282,236 as well as in U.S. Patent 4,879,288, the entire contents of which are herein incorporated by reference.

### EXAMPLE 1

The following process was used to prepare tablets having the composition defined in Table 1.

11-[4-[2-(2-hydroxyethoxy)ethyl]-1-piperazinyl]-dibenzo-[b,f]]1,4]thiazepine hemifumurate (3453.8g), lactose (1144.7g), microcrystalline cellulose (381.5g) and METHOCEL® E50LV (900g) were blended in a planetary mixer for approximately 3 minutes.

The mixture was wet granulated in a planetary mixer using purified water. The wet mass was dried in a fluidized bed drier at about 65°C until the loss on drying was less than about 3% as measured by a moisture balance.

The dried granulation was milled using a hammer type or similar mill operating at fast speed, knives forward with suitable screen (e.g. 20 to 40 mesh).

Magnesium stearate was passed through an appropriate screen (e.g. 20 to 40 mesh).

The dry granulated material was blended for approximately 3 minutes in a conventional blender (for example, Patterson-Kelley Twin Shell) with the screened magnesium stearate.

The blended mixture was compressed into tablets using a conventional rotary tablet press (for example, Kilian LX-21).

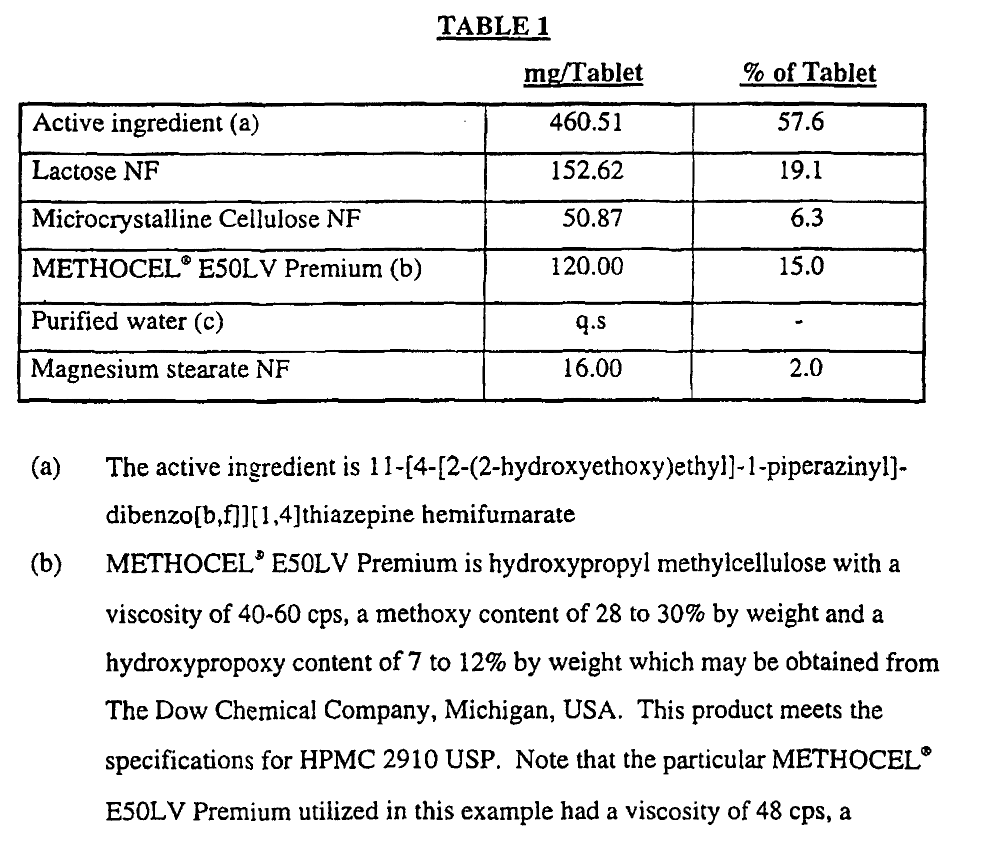

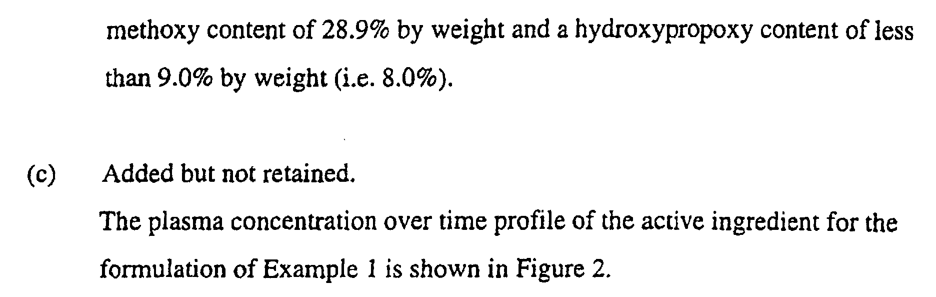

### EXAMPLE 2

The procedure described in Example 1 was repeated using METHOCEL® E50LV and METHOCEL® E4M in place of METHOCEL® E50LV to afford tablets of the following composition.

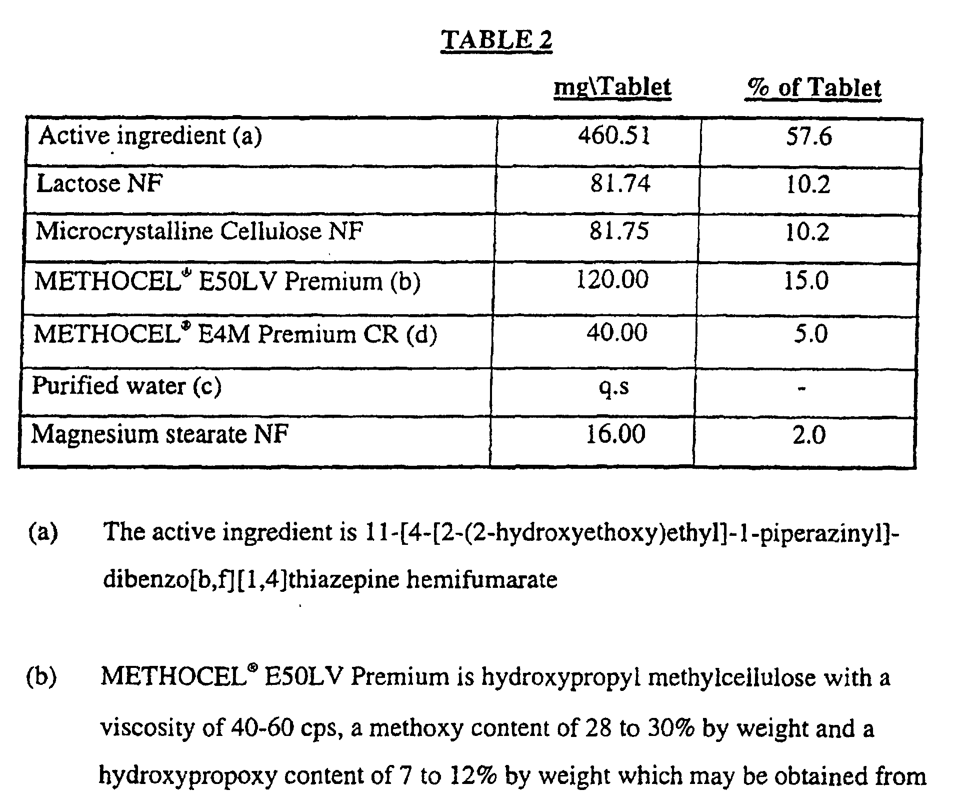

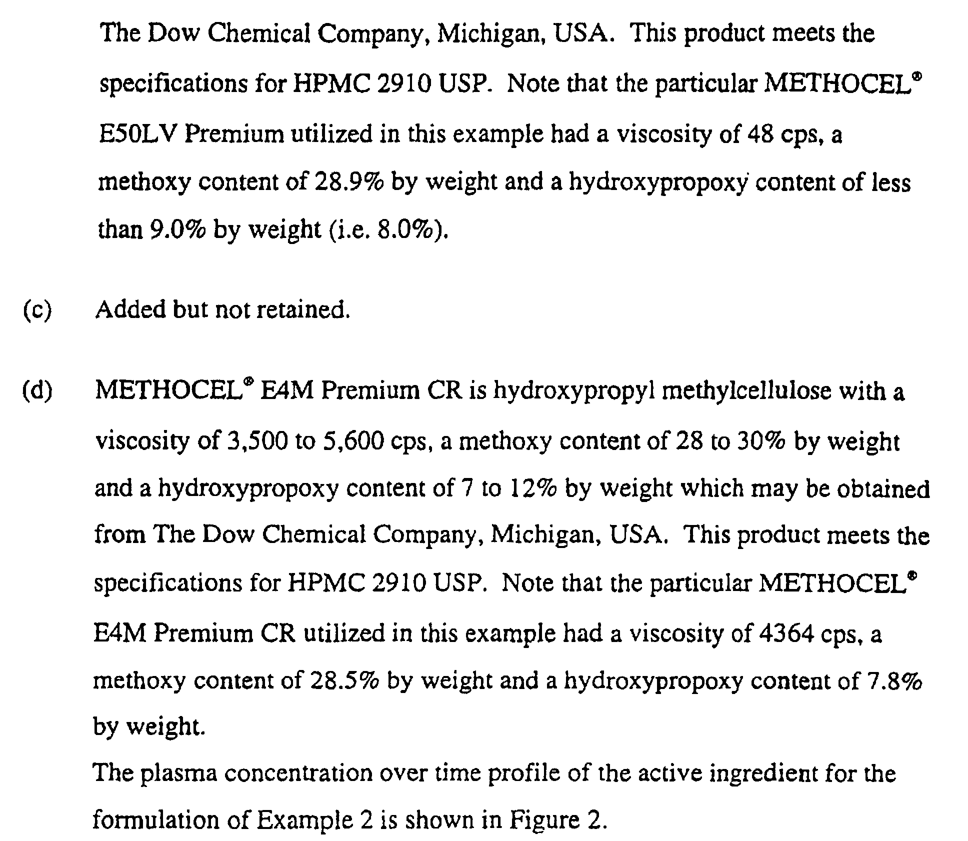

### Example 3

Following a procedure similar to that described in Example 1, tablets of the following composition can be prepared.

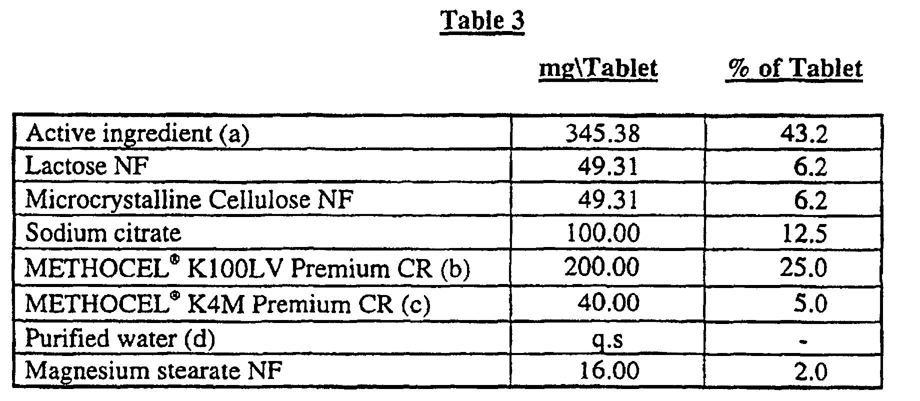

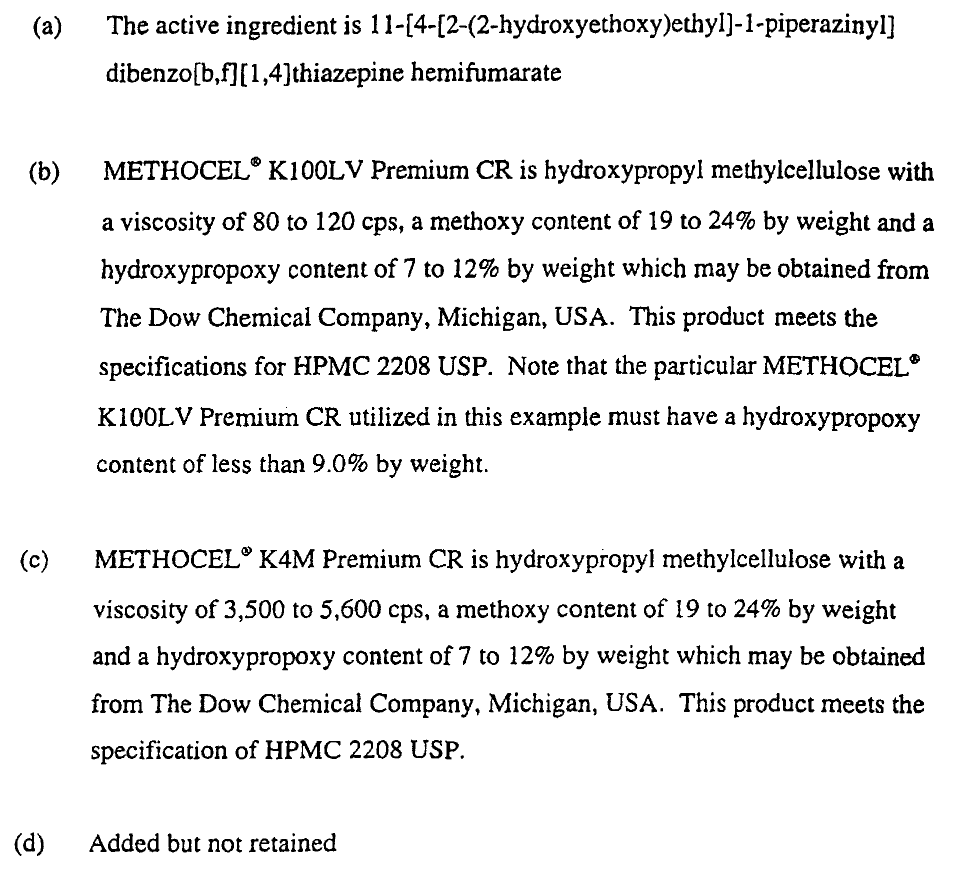

### Example 4

Following a procedure similar to that described in Example 1, tablets of the following composition can be prepared.

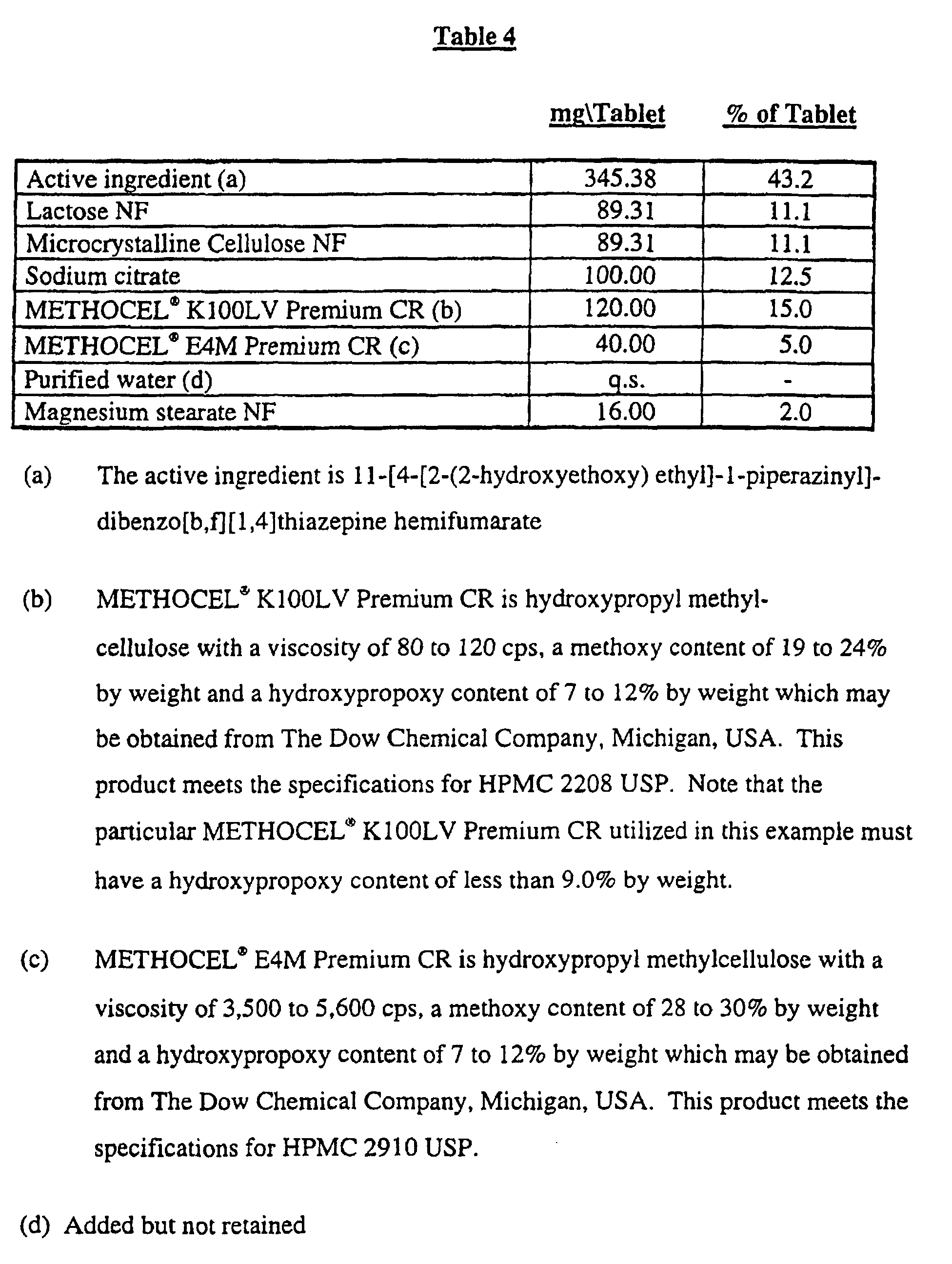

### Example 5

Following a procedure similar to that described in Example 1, tablets of the following composition can be prepared.

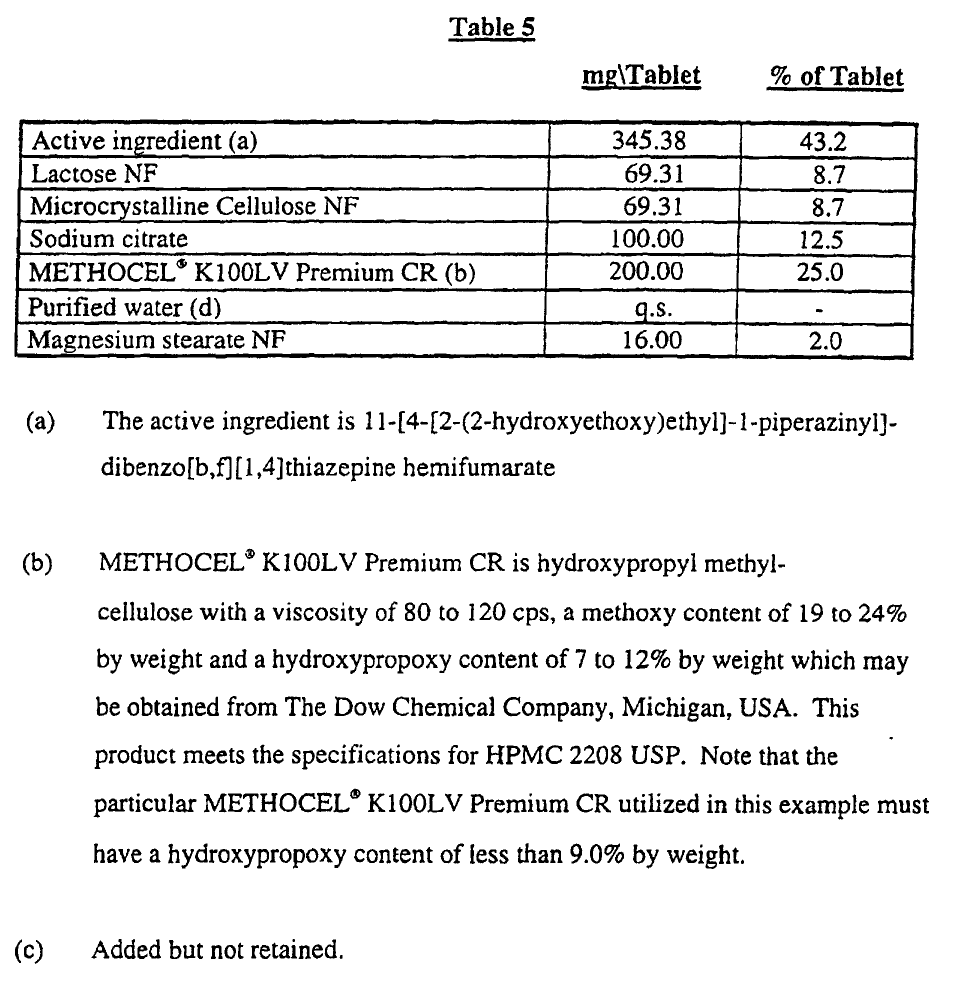

### Example 6

Following a procedure similar to that described in Example 1, tablets of the following composition can be prepared.

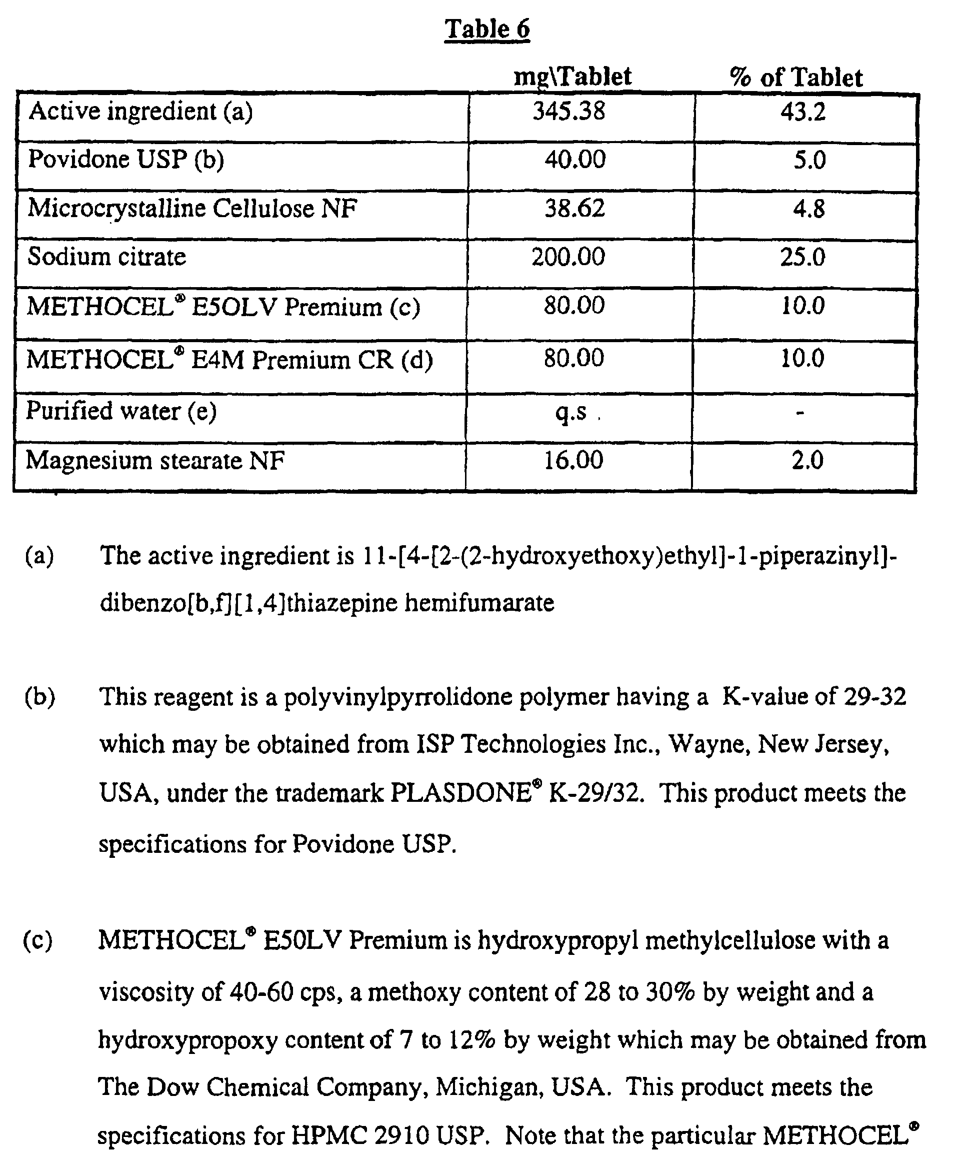

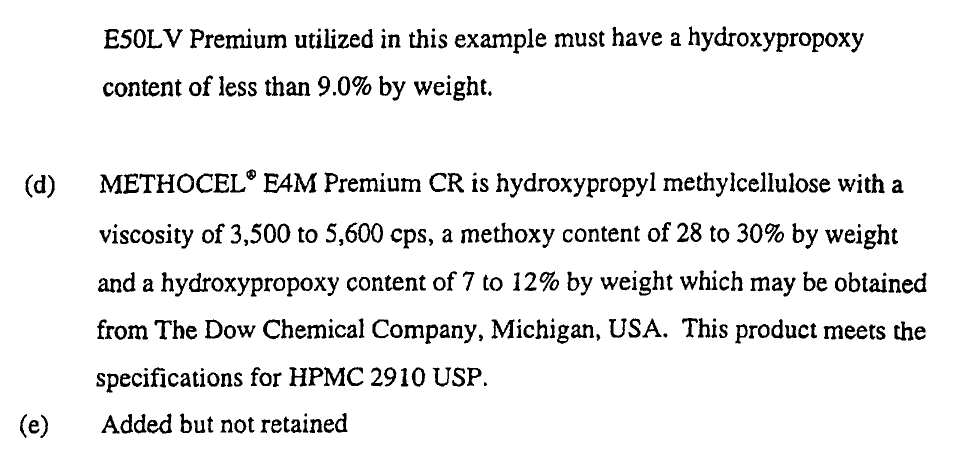

### Example 7

Following a procedure similar to that described in Example 1, tablets of the following composition can be prepared.

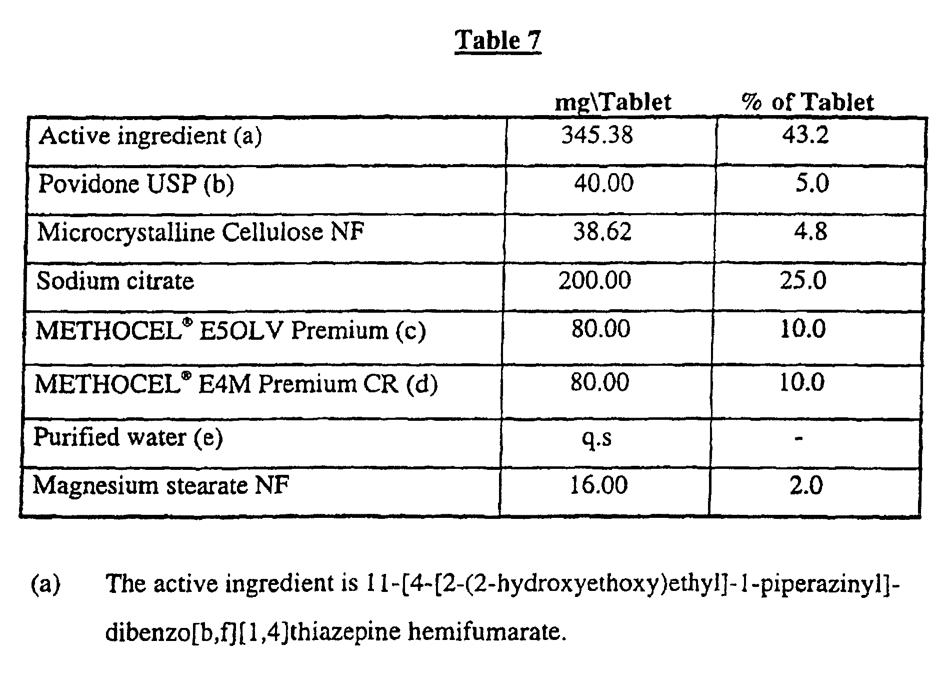

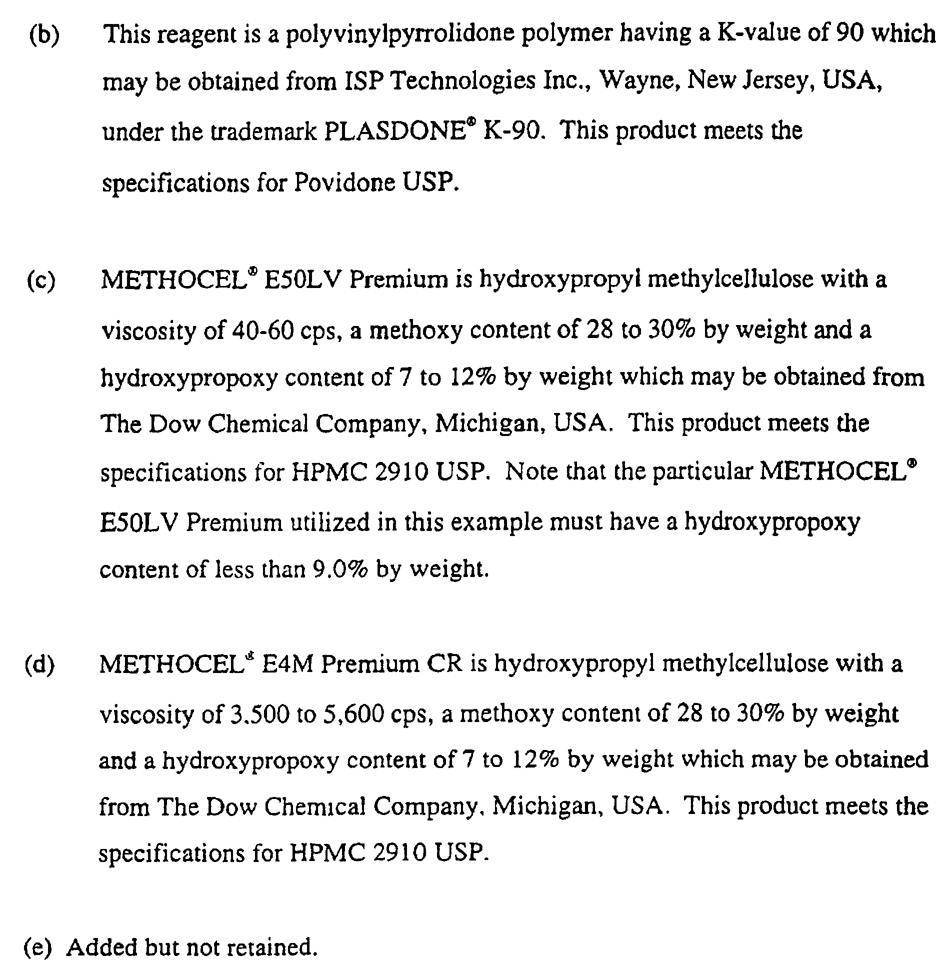

Following a procedure similar to that described in Example 1, tablets of the following compositions were prepared:

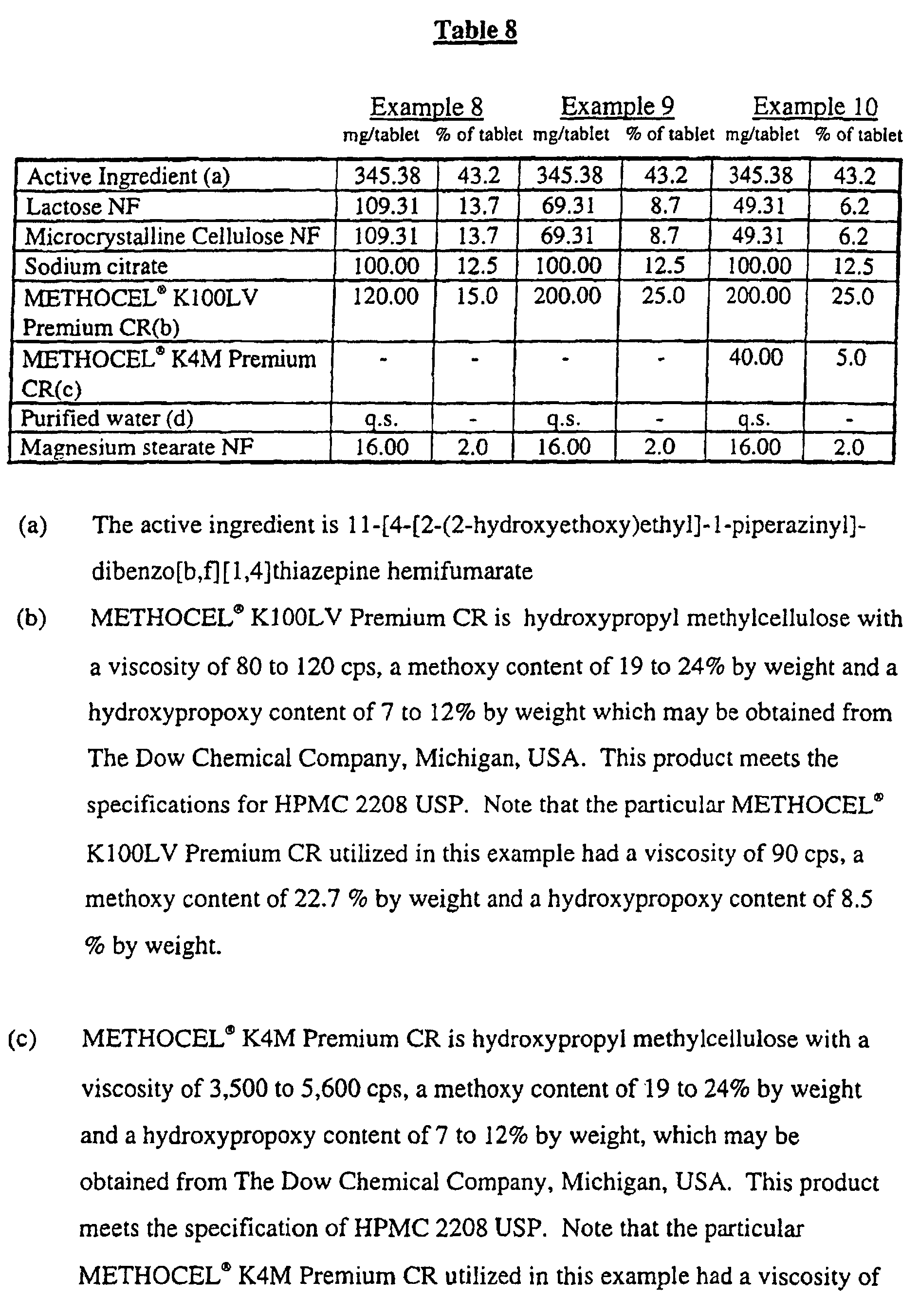

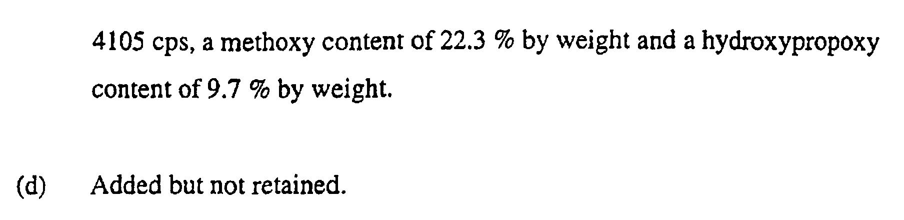

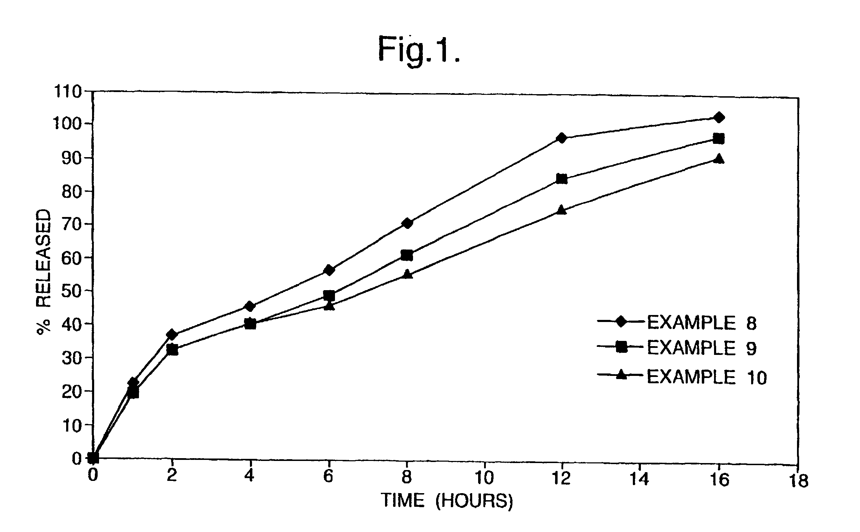

The release dissolution profile of the formulations of Examples 8, 9 and 10 are shown in Figure 1.

### Example 11

Following a procedure similar to that described in Example 1, tablets of the following composition were prepared:

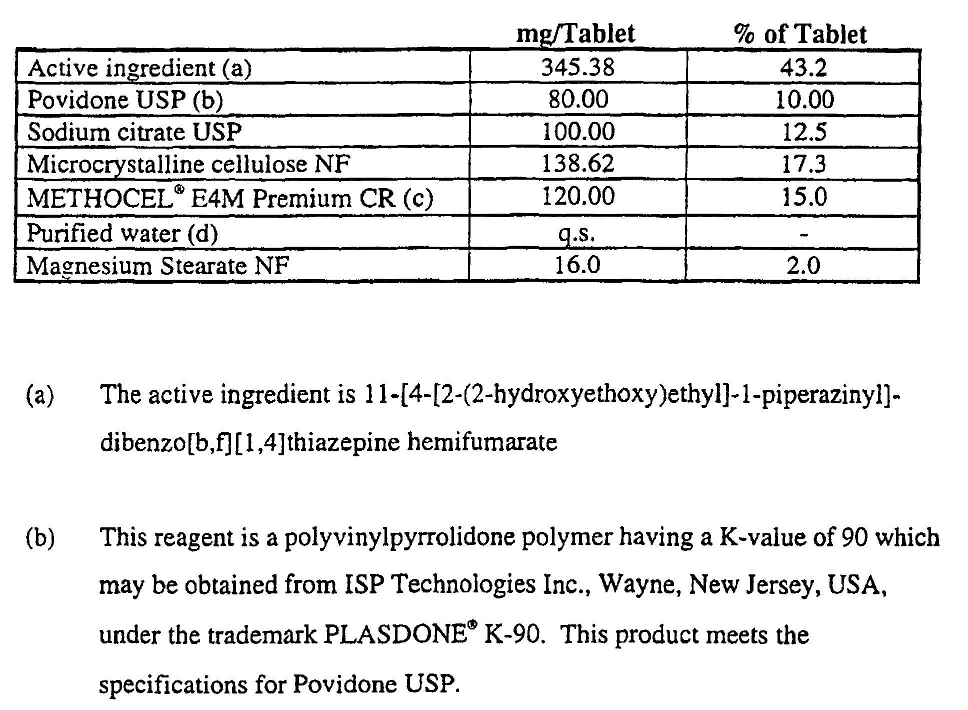

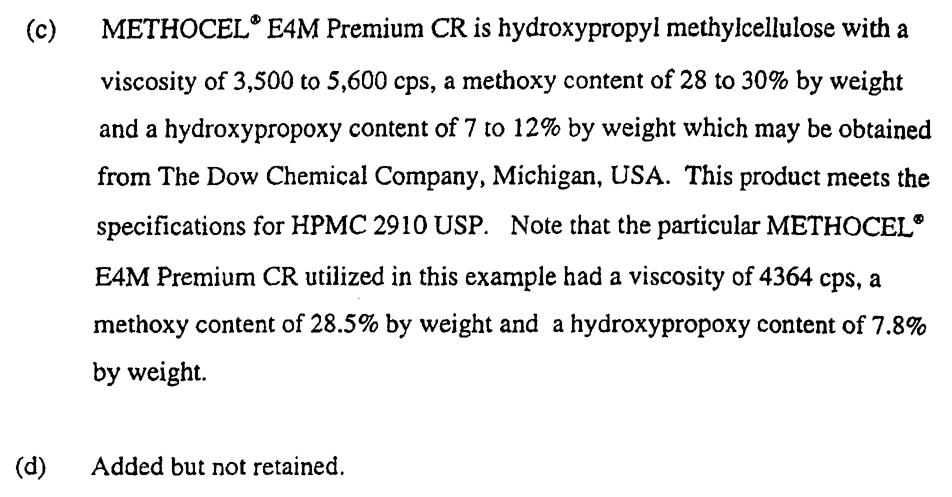

### Example 12

| **CORE** | **Mg/Tablet** |
|---|---|
| Active ingredient (a) | 115.13 |
| Povidone USP (b) | 8.33 |
| Dicalcium phosphate dihydrate USP | 10.00 |
| Microcrystalline cellulose NF | 32.88 |
| Sodium starch glycolate NF | 8.33 |
| Lactose NF | 22.33 |
| Magnesium stearate NF | 3.00 |
| Purified water (c) | q.s. |

| **COATING** | **Mg/Tablet** |
|---|---|
| Hydroxypropyl methylcellulose 2910 USP (d) | 5.00 |
| Polyethylene glycol 400 NF | 1.00 |
| Yellow ferric oxide NF | 0.15 |
| Titanium dioxide USP | 1.85 |

| | |
|---|---|
| (a) The active ingredient is 11-[4-[2-(2-hydroxyethoxy)ethyl]-1-piperazinyl]dibenzo[b,f][1,4]thiazepine hemifumarate. | |
| (b) This reagent is a polyvinylpyrrolidone polymer having a K-value of 29-32 which may be obtained from ISP Technologies Inc., Wayne, New Jersey, USA, under the trademark PLASDONE® K-29/32. This product meets the specification for Povidone USP. | |
| (c) Added but not retained. | |
| (d) The hydroxypropyl methylcellulose utilized in this example was PHARMACOAT® 606 which may be obtained from Shin-Etsu, Ltd., Japan and has a viscosity in the range of 4.5 to 8.0 cps, a methoxy content of 28 to 30% by weight and a hydroxypropoxy content of 7 to 12 % by weight. | |

The above described immediate release composition was prepared by the following process: The active ingredient, povidone, dicalcium phosphate dihydrate, and portions of the microcrystalline cellulose and sodium starch glycolate were mixed in a mixer-granulator (for example, a Littleford MGT) for approximately 5 minutes. Purified water was added while mixing until a suitable mass was obtained. The wet granules were passed through a cone mill fitted with an appropriate screen (e.g. 6.35 mm) and then were dried in a fluidized bed dryer set at an inlet temperature of approximately 65°C to a loss on drying level of less than 2.5% w/w. The dried granules were then passed through a suitable mill fitted with an appropriate screen (e.g. #20 mesh in a hammer mill). The granulation was combined in a blender (e.g. V-blender) with lactose and the remainder of the microcrystalline cellulose and sodium starch glycolate and was blended for approximately 5 minutes. The magnesium stearate was passed through a suitable mill fitted with an appropriate screen (e.g. 40 mesh) and then was added to the dry granulated material and blended for approximately 3 minutes. The blended mixture was then compressed into tablets using conventional rotary compression equipment. The tablets were then film coated using conventional drum coating equipment with an aqueous suspension of the film coating constituents (i.e. hydroxypropyl methylcellulose, polyethylene glycol 400, yellow ferric oxide and titanium dioxide) at an inlet temperature of approximately 80°C.

## Claims

1. A sustained release formulation comprising a gelling agent and 11-[4-[2-(2-hydroxyethoxy)ethyl]-1-piperazinyl]dibenzo-[b,f][1,4]thiazepine or a pharmaceutically acceptable salt thereof, together with one or more pharmaceutically acceptable excipients.

2. A sustained release formulation according to claim 1 such that 11-[4-[2-(2-hydroxyethoxy)ethyl]-1-piperazinyl]dibenzo-[b,f][1,4]thiazepine or a pharmaceutically acceptable salt thereof is released from the formulation, in a controlled fashion over a period of between 8 and 24 hours so that at least 60% of 11-[4-[2-(2-hydroxyethoxy)ethyl]-1-piperazinyl]dibenzo-[b,f][1,4]thiazepine or a pharmaceutically acceptable salt thereof has been released at the end of this period.

3. A sustained release formulation according to claim 1 or claim 2 wherein the gelling agent is hydroxypropyl methylcellulose.

4. A sustained release formulation according to claim 3 comprising about 5 to 50% by weight of a hydroxypropyl methylcellulose selected from the group consisting of (a) a hydroxypropyl methylcellulose having a viscosity of about 40 to 60 cps, a methoxy content of about 28 to 30% by weight and a hydroxypropoxy content of from about 7 to less than 9% by weight, (b) a hydroxypropyl methylcellulose having a viscosity of about 3,500 to 5,600 cps, a methoxy content of about 28 to 30% by weight and a hydroxypropoxy content of about 7 to 12% by weight, (c) a hydroxypropyl methylcellulose having a viscosity of about 80 to 120 cps, a methoxy content of about 19 to 24% by weight and a hydroxypropoxy content of from about 7 to less than 9% by weight and (d) a hydroxypropyl methylcellulose having a viscosity of about 3,500 to 5,600 cps, a methoxy content of about 19 to 24% by weight and a hydroxypropoxy content of about 7 to 12% by weight, or mixtures thereof.

5. A sustained release formulation according to claim 3 comprising about 5 to 50% by weight of a hydroxypropyl methylcellulose selected from the group consisting of (a) a hydroxypropyl methylcellulose having a viscosity of about 40 to 60 cps, a methoxy content of about 28 to 30% by weight and a hydroxypropoxy content of from about 7 to less than 9% by weight, (b) a hydroxypropyl methylcellulose having a viscosity of about 3,500 to 5,600 cps, a methoxy content of about 28 to 30% by weight and a hydroxypropoxy content of about 7 to 12% by weight, (c) a hydroxypropyl methylcellulose having a viscosity of about 80 to 120 cps, a methoxy content of about 19 to 24% by weight and a hydroxypropoxy content of from about 7 to less than 9% by weight and (d) a hydroxypropyl methylcellulose having a viscosity of about 3,500 to 5,600 cps, a methoxy content of about 19 to 24% by weight and a hydroxypropoxy content of about 7 to 12% by weight, or mixtures thereof with the proviso that if the formulation contains a hydroxypropyl methylcellulose described under (d) above the total amount of hydroxypropyl methylcellulose present in the formulation must be greater than 25.8% by weight.

6. A sustained release formulation according to claim 4 or claim 5 comprising about 5 to 40% by weight of a hydroxypropyl methylcellulose selected from the group consisting of (a) - (d) or mixtures thereof.

7. A sustained release formulation according to claim 6 comprising about 8 to 35% by weight of a hydroxypropyl methylcellulose selected from the group consisting of (a) - (d) or mixtures thereof.

8. A formulation according to claim 7 comprising about 10 to 30% by weight of a hydroxypropyl methylcellulose selected from the groups (a) - (d) or mixtures thereof.

9. A formulation according to claim 8 comprising about 15 to 30% by weight of a hydroxypropyl methylcellulose selected from the groups (a) - (d) or mixtures thereof.

10. A formulation according to anyone of claims 1-9 wherein 11-[4-[2-(2-hydroxyethoxy)ethyl]-1-piperazinyl]dibenzo[b,f][1,4]thiazepine or a pharmaceutically acceptable salt thereof is present in about 35 to 65% by weight.

11. A formulation according to claim 10 wherein the amount of hydroxypropyl methylcellulose is about 5 to 40%.

12. A formulation according to claims 1-11 wherein the one or more pharmaceutically acceptable excipients are selected from the group consisting of microcrystalline cellulose, lactose, magnesium stearate, sodium citrate and povidone.

13. A formulation according to claim 12 wherein the one or more pharmaceutically acceptable excipients are selected from the group consisting of (a) about 4 to 20% by weight of microcrystalline cellulose, (b) about 5 to 20% by weight of lactose, (c) about 1 to 3% by weight of magnesium stearate, (d) about 10 to 30% by weight of sodium citrate and (e) about 1 to 15% by weight of povidone.

14. A formulation according to anyone of claims 1-13 wherein one of the one or more pharmaceutically acceptable excipients is a pH modifier.

15. A formulation according to claim 14 wherein the pH modifier is sodium citrate.

16. A formulation according to any of claims 1-15 wherein 11-[4-[2-(2-hydroxyethoxy)ethyl]-1-piperazinyl]dibenzo[b,f][1,4]thiazepine is in the form of a hemifumarate salt.

17. A formulation according to any one of claims 1-16 wherein the formulation is coated.

18. The use of a formulation according to any one of claims 1-17 in the manufacture of a medicament for treating psychotic states or hyperactivity in a warm-blooded animal.

19. A process for preparing a formulation according to any one of claims 1-17 which comprises mixing 11-[4-[2-(2-hydroxyethoxy)ethyl]-1-piperazinyl]-dibenzo[b,f][1,4]thiazepine, or a pharmaceutically acceptable salt thereof, a gelling agent and other excipients.

20. A process for preparing a formulation according to any one of claims 1-17 which comprises:
(a) mixing 11-[4-[2-(2-hydroxyethoxy)ethyl]-1-piperazinyl]-dibenzo[b,f][1,4]thiazepine, or a pharmaceutically acceptable salt thereof, a gelling agent and other excipients;
(b) wet granulating the mixed components;
(c) drying the mixture;
(d) milling the dried mixture;
(e) blending the mixture with a lubricant; and
(f) compressing the blended mixture to form tablets, and optionally coating said tablets.

## Patentansprüche

1. Retard-Formulierung, enthaltend ein Geliermittel und 11-[4-[2-(2-Hydroxyethoxy)ethyl]-1-piperazinyl]dibenzo-[b,f][1,4]thiazepin oder ein pharmazeutisch unbedenkliches Salz davon zusammen mit einem oder mehreren pharmazeutisch unbedenklichen Hilfsstoffen.

2. Retard-Formulierung nach Anspruch 1, die so beschaffen ist, daß 11-[4-[2-(2-Hydroxyethoxy)ethyl]-1-piperazinyl]dibenzo-[b,f][1,4]thiazepin oder ein pharmazeutisch unbedenkliches Salz davon in kontrollierter Weise über einen Zeitraum von 8 bis 24 Stunden aus der Formulierung freigesetzt wird, so daß am Ende dieses Zeitraums wenigstens 60% des 11-[4-[2-(2-Hydroxyethoxy)ethyl]-1-piperazinyl]dibenzo-[b,f]-[1,4]thiazepin bzw. eines pharmazeutisch unbedenklichen Salzes davon freigesetzt worden sind.

3. Retard-Formulierung nach Anspruch 1 oder 2, wobei es sich bei dem Geliermittel um Hydroxypropylmethylcellulose handelt.

4. Retard-Formulierung nach Anspruch 3, enthaltend etwa 5 bis 50 Gew.-% einer Hydroxypropylmethylcellulose ausgewählt aus der Gruppe bestehend aus (a) einer Hydroxypropylmethylcellulose mit einer Viskosität von etwa 40 bis 60 cps, einem Methoxygehalt von etwa 28 bis 30 Gew.-% und einem Hydroxypropoxygehalt von etwa 7 bis weniger als 9 Gew.-%, (b) einer Hydroxypropylmethylcellulose mit einer Viskosität von etwa 3500 bis 5600 cps, einem Methoxygehalt von etwa 28 bis 30 Gew.-% und einem Hydroxypropoxygehalt von etwa 7 bis 12 Gew.-%, (c) einer Hydroxypropylmethylcellulose mit einer Viskosität von etwa 80 bis 120 cps, einem Methoxygehalt von etwa 19 bis 24 Gew.-% und einem Hydroxypropoxygehalt von etwa 7 bis weniger als 9 Gew.-% und (d) einer Hydroxypropylmethylcellulose mit einer Viskosität von etwa 3500 bis 5600 cps, einem Methoxygehalt von etwa 19 bis 24 Gew.-% und einem Hydroxypropoxygehalt von etwa 7 bis 12 Gew.-%, oder Mischungen davon.

5. Retard-Formulierung nach Anspruch 3, enthaltend etwa 5 bis 50 Gew.-% einer Hydroxypropylmethylcellulose ausgewählt aus der Gruppe bestehend aus (a) einer Hydroxypropylmethylcellulose mit einer Viskosität von etwa 40 bis 60 cps, einem Methoxygehalt von etwa 28 bis 30 Gew.-% und einem Hydroxypropoxygehalt von etwa 7 bis weniger als 9 Gew.-%, (b) einer Hydroxypropylmethylcellulose mit einer Viskosität von etwa 3500 bis 5600 cps, einem Methoxygehalt von etwa 28 bis 30 Gew.-% und einem Hydroxypropoxygehalt von etwa 7 bis 12 Gew.-%, (c) einer Hydroxypropylmethylcellulose mit einer Viskosität von etwa 80 bis 120 cps, einem Methoxygehalt von etwa 19 bis 24 Gew.-% und einem Hydroxypropoxygehalt von etwa 7 bis weniger als 9 Gew.-% und (d) einer Hydroxypropylmethylcellulose mit einer Viskosität von etwa 3500 bis 5600 cps, einem Methoxygehalt von etwa 19 bis 24 Gew.-% und einem Hydroxypropoxygehalt von etwa 7 bis 12 Gew.-%, oder Mischungen davon, mit der Maßgabe, daß, wenn die Formulierung eine oben unter (d) beschriebene Hydroxypropylmethylcellulose enthält, die Gesamtmenge an in der Formulierung vorliegender Hydroxypropylmethylcellulose mehr als 25,8 Gew.-% betragen muß.

6. Retard-Formulierung nach Anspruch 4 oder 5, enthaltend etwa 5 bis 40 Gew.-% einer aus der aus (a) - (d) oder Mischungen davon bestehenden Gruppe ausgewählten Hydroxypropylmethylcellulose.

7. Retard-Formulierung nach Anspruch 6, enthaltend etwa 8 bis 35 Gew.-% einer aus der aus (a) - (d) oder Mischungen davon bestehenden Gruppe ausgewählten Hydroxypropylmethylcellulose.

8. Formulierung nach Anspruch 7, enthaltend etwa 10 bis 30 Gew.-% einer aus der aus (a) - (d) oder Mischungen davon bestehenden Gruppe ausgewählten Hydroxypropylmethylcellulose.

9. Formulierung nach Anspruch 8, enthaltend etwa 15 bis 30 Gew.-% einer aus der aus (a) - (d) oder Mischungen davon bestehenden Gruppe ausgewählten Hydroxypropylmethylcellulose.

10. Formulierung nach einem der Ansprüche 1-9, wobei 11-[4-[2-(2-Hydroxyethoxy)ethyl]-1-piperazinyl]-dibenzo-[b,f][1,4]thiazepin oder ein pharmazeutisch unbedenkliches Salz davon in einer Menge von etwa 35 bis 65 Gew.-% vorliegt.

11. Formulierung nach Anspruch 10, wobei die Menge an Hydroxypropylmethylcellulose etwa 5 bis 40% beträgt.

12. Formulierung nach Ansprüchen 1-11, wobei das eine bzw. die mehreren pharmazeutisch unbedenklichen Hilfsstoffe aus der aus mikrokristalliner Cellulose, Lactose, Magnesiumstearat, Natriumcitrat und Povidon bestehenden Gruppe ausgewählt ist bzw. sind.

13. Formulierung nach Anspruch 12, wobei das eine bzw. die mehreren pharmazeutisch unbedenklichen Hilfsstoffe aus der aus (a) etwa 4 bis 20 Gew.-% mikrokristalliner Cellulose, (b) etwa 5 bis 20 Gew.-% Lactose, (c) etwa 1 bis 3 Gew.-% Magnesiumstearat, (d) etwa 10 bis 30 Gew.-% Natriumcitrat und (e) etwa 1 bis 15 Gew.-% Povidon bestehenden Gruppe ausgewählt sind.

14. Formulierung nach einem der Ansprüche 1-13, wobei es sich bei einem der einen oder mehreren pharmazeutisch unbedenklichen Hilfsstoffe um ein Mittel zur Modifizierung des pH-Wertes handelt.

15. Formulierung nach Anspruch 14, wobei es sich bei dem Mittel zur Modifizierung des pH-Wertes um Natriumcitrat handelt.

16. Formulierung nach einem der Ansprüche 1-15, wobei das 11-[4-[2-(2-Hydroxyethoxy)ethyl]-1-piperazinyl]dibenzo-[b,f][1,4]thiazepin in Form eines Hemifumaratsalzes vorliegt.

17. Formulierung nach einem der Ansprüche 1-16, wobei die Formulierung überzogen ist.

18. Verwendung einer Formulierung nach einem der Ansprüche 1-17 bei der Herstellung eines Medikaments zur Behandlung von psychotischen Zuständen oder Hyperaktivität bei einem Warmblüter.

19. Verfahren zur Herstellung einer Formulierung nach einem der Ansprüche 1-17, bei dem man 11-[4-[2-(2-Hydroxyethoxy]ethyl]-1-piperazinyl]dibenzo-[b,f][1,4]thiazepin oder ein pharmazeutisch unbedenkliches Salz davon, ein Geliermittel und andere Hilfsstoffe mischt.

20. Verfahren zur Herstellung einer Formulierung nach einem der Ansprüche 1-17, bei dem man:
(a) 11-[4-[2-(2-Hydroxyethoxy)ethyl]-1-piperazinyl]dibenzo-[b,f][1,4]thiazepin oder ein pharmazeutisch unbedenkliches Salz davon, ein Geliermittel und andere Hilfsstoffe mischt;
(b) die gemischten Komponenten naß granuliert;
(c) die Mischung trocknet;
(d) die getrocknete Mischung mahlt;
(e) die Mischung mit einem Gleitmittel vermengt; und
(f) die vermengte Mischung zu Tabletten verpreßt und die Tabletten gegebenenfalls überzieht.

## Revendications

1. Formulation à libération prolongée comprenant un agent gélifiant et de la 11-[4-[2-(2-hydroxyéthoxy)éthyl]-1-pipérazinyl]dibenzo-[b,f][1,4]thiazépine ou un sel pharmaceutiquement acceptable de celle-ci, conjointement avec un ou plusieurs excipients pharmaceutiquement acceptables.

2. Formulation à libération prolongée selon la revendication 1, de telle sorte que la 11-[4-[2-(2-hydroxyéthoxy)éthyl]-1-pipérazinyl]dibenzo-[b,f][1,4]thiazépine ou un sel pharmaceutiquement acceptable de celle-ci est libéré(e) de la formulation, de façon régulée sur une période comprise entre 8 et 24 heures, si bien qu'au moins 60 % de la 11-[4-[2-(2-hydroxyéthoxy)éthyl]-l-pipérazinyl]dibenzo-[b,f][1,4]thiazépine ou un sel pharmaceutiquement acceptable de celle-ci ont été libérés à la fin de cette période.

3. Formulation à libération prolongée selon la revendication 1 ou la revendication 2, dans laquelle l'agent gélifiant est l'hydroxypropylméthylcellulose.

4. Formulation à libération prolongée selon la revendication 3, comprenant d'environ 5 à 50 % en poids d'une hydroxypropylméthylcellulose choisie parmi le groupe constitué de (a) une hydroxypropylméthylcellulose ayant une viscosité d'environ 40 à 60 cps, une teneur en méthoxy d'environ 28 à 30 % en poids et une teneur en hydroxypropoxy d'environ 7 à moins de 9 % en poids, (b) une hydroxypropylméthylcellulose ayant une viscosité d'environ 3 500 à 5 600 cps, une teneur en méthoxy d'environ 28 à 30 % en poids et une teneur en hydroxypropoxy d'environ 7 à 12 % en poids, (c) une hydroxypropylméthylcellulose ayant une viscosité d'environ 80 à 120 cps, une teneur en méthoxy d'environ 19 à 24 % en poids et une teneur en hydroxypropoxy d'environ 7 à moins de 9 % en poids et (d) une hydroxypropylméthylcellulose ayant une viscosité d'environ 3 500 à 5 600 cps, une teneur en méthoxy d'environ 19 à 24 % en poids et une teneur en hydroxypropoxy d'environ 7 à 12 % en poids, ou leurs mélanges.

5. Formulation à libération prolongée selon la revendication 3, comprenant d'environ 5 à 50 % en poids d'une hydroxypropylméthylcellulose choisie parmi le groupe constitué de (a) une hydroxypropylméthylcellulose ayant une viscosité d'environ 40 à 60 cps, une teneur en méthoxy d'environ 28 à 30 % en poids et une teneur en hydroxypropoxy d'environ 7 à moins de 9 % en poids, (b) une hydroxypropylméthylcellulose ayant une viscosité d'environ 3 500 à 5 600 cps, une teneur en méthoxy d'environ 28 à 30 % en poids et une teneur en hydroxypropoxy d'environ 7 à 12 % en poids, (c) une hydroxypropylméthylcellulose ayant une viscosité d'environ 80 à 120 cps, une teneur en méthoxy d'environ 19 à 24 % en poids et une teneur en hydroxypropoxy d'environ 7 à moins de 9 % en poids et (d) une hydroxypropylméthylcellulose ayant une viscosité d'environ 3 500 à 5 600 cps, une teneur en méthoxy d'environ 19 à 24 % en poids et une teneur en hydroxypropoxy d'environ 7 à 12 % en poids, ou leurs mélanges, à condition que si la formulation contient une hydroxypropylméthylcellulose décrite en (d) ci-dessus, la quantité totale d'hydroxypropylméthylcellulose présente dans la formulation doit être supérieure à 25,8 % en poids.

6. Formulation à libération prolongée selon la revendication 4 ou la revendication 5, comprenant d'environ 5 à 40 % en poids d'une hydroxypropylméthylcellulose choisie parmi le groupe constitué de (a) à (d) ou de leurs mélanges.

7. Formulation à libération prolongée selon la revendication 6, comprenant d'environ 8 à 35 % en poids d'une hydroxypropylméthylcellulose choisie parmi le groupe constitué de (a) à (d) ou de leurs mélanges.

8. Formulation selon la revendication 7, comprenant d'environ 10 à 30 % en poids d'une hydroxypropylméthylcellulose choisie parmi le groupe constitué de (a) à (d) ou de leurs mélanges.

9. Formulation selon la revendication 8, comprenant d'environ 15 à 30 % en poids d'une hydroxypropylméthylcellulose choisie parmi le groupe constitué de (a) à (d) ou de leurs mélanges.

10. Formulation selon l'une quelconque des revendications 1 à 9, dans laquelle la 11-[4-[2-(2-hydroxyéthoxy)éthyl]-1-pipérazinyl]dibenzo-[b,f][1,4]thiazépine ou un sel pharmaceutiquement acceptable de celle-ci est présent(e) à environ 35 à 65 % en poids.

11. Formulation selon la revendication 10, dans laquelle la quantité d'hydroxypropylméthylcellulose est d'environ 5 à 40 %.

12. Formulation selon les revendications 1 à 11, dans laquelle l'un ou plusieurs excipients pharmaceutiquement acceptables sont choisis parmi le groupe constitué de la cellulose microcristalline, du lactose, du stéarate de magnésium, du citrate de sodium et de la povidone.

13. Formulation selon la revendication 12, dans laquelle l'un ou plusieurs excipients pharmaceutiquement acceptables sont choisis parmi le groupe constitué de (a) environ 4 à 20% en poids de cellulose microcristalline, (b) environ 5 à 20% en poids de lactose, (c) environ 1 à 3 % en poids de stéarate de magnésium, (d) environ 10 à 30 % en poids de citrate de sodium et (e) environ 1 à 15 % en poids de povidone.

14. Formulation selon l'une quelconque des revendications 1 à 13, dans laquelle l'un parmi l'un ou plusieurs excipients pharmaceutiquement acceptables est un modificateur de pH.

15. Formulation selon la revendication 14, dans laquelle le modificateur de pH est le citrate de sodium.

16. Formulation selon l'une quelconque des revendications 1 à 15, dans laquelle la 11-[4-[2-(2-hydroxyéthoxy)éthyl]-1-pipérazinyl]dibenzo-[b,f][1,4]thiazépine se trouve sous la forme d'un sel d'hémifumarate.

17. Formulation selon l'une quelconque des revendications 1 à 16, dans laquelle la formulation est enrobée.

18. Utilisation d'une formulation selon l'une quelconque des revendications 1 à 17, pour l'élaboration d'un médicament destiné à traiter des états psychotiques ou une hyperactivité chez un animal à sang chaud.

19. Procédé de préparation d'une formulation selon l'une quelconque des revendications 1 à 17, comprenant la mixtion de la 11-[4-[2- (2-hydroxyéthoxy)éthyl]-1-pipérazinyl]dibenzo-[b, f][1,4]thiazépine ou d'un sel pharmaceutiquement acceptable de celle-ci, d'un agent gélifiant et d'autres excipients.

20. Procédé de préparation d'une formulation selon l'une quelconque des revendications 1 à 17, comprenant les étapes consistant à :
(a) mélanger la 11-[4-[2-(2-hydroxyéthoxy)éthyl]-1-pipérazinyl]dibenzo-[b,f][1,4]thiazépine ou un sel pharmaceutiquement acceptable de celle-ci, un agent gélifiant et d'autres excipients ;
(b) granuler les composants mélangés à l'état mouillé ;
(c) sécher le mélange ;
(d) broyer le mélange séché ;
(e) homogénéiser le mélange avec un lubrifiant ; et
(f) comprimer le mélange homogénéisé en vue de former des comprimés, et éventuellement enrober lesdits comprimés.
